Europäisches Patentamt

European Patent Office

Office européen des brevets

(11) Publication number: **0 060 141**
**B1**

(12) **EUROPEAN PATENT SPECIFICATION**

(45) Date of publication of patent specification: **05.11.86**

(51) Int. Cl.⁴: **A 61 K 37/26**

(21) Application number: **82301207.5**

(22) Date of filing: **09.03.82**

(54) Stabilized insulin preparations and method for their production.

(30) Priority: **10.03.81 GB 8107423**
**18.09.81 DK 4143/81**

(43) Date of publication of application:
**15.09.82 Bulletin 82/37**

(45) Publication of the grant of the patent:
**05.11.86 Bulletin 86/45**

(84) Designated Contracting States:
**AT DE NL SE**

(56) References cited:
**FR-A- 892 194**
**FR-M- 662**
**US-A-3 102 077**
**US-A-4 196 196**

**CHEMICAL ABSTRACTS, vol. 89, 1978, page
343, no. 39950x, Columbus, Ohio, USA, s.L.
HOWELL et al.: "Role of zinc and calcium in the
formation and storage of insulin in the
pancreatic beta-cell"**

The file contains technical information
submitted after the application was filed and
not included in this specification

(73) Proprietor: **NOVO INDUSTRI A/S**
**Novo Allé**
**DK-2880 Bagsvaerd (DK)**

(72) Inventor: **Brange, Jens Jorgen Veilgaard**
**22a Kroyersvej**
**DK-2930 Klampenborg (DK)**
Inventor: **Havelund, Svend**
**38 Bavnevej**
**DK-2650 Hvidovre (DK)**

(74) Representative: **Brown, John David et al**
**FORRESTER & BOEHMERT Widenmayerstrasse
4/I**
**D-8000 München 22 (DE)**

(56) References cited:
**CHEMICAL ABSTRACTS, vol. 91, 1979, page
218, no. 15481k, Columbus, Ohio, USA, J.
HUDECEK et al.: "Interaction of insulin with
metal (II) ions"**

Courier Press, Leamington Spa, England.

## Description

The present invention relates to novel stabilized insulin preparations which are specifically adapted for use in equipment for continuous insulin delivery, and to a process for producing such stabilized insulin preparations.

Over the past years steadily increasing efforts have been devoted to the development of portable or implantable systems for continuous infusion of insulin, the main aim of such efforts being to put at the disposal of the diabetic patient a regime of insulin administration which is more closely adaptable to diurnal variations in his physiological insulin requirements than is possible by conventional insulin medication.

In essence, the mechanical part of a device for continuous insulin delivery comprises such elements as an insulin reservoir, a pumping system and a suitable catheter for delivering the insulin at the chosen site, which is usually located either subcutaneously, intravenously, or in the peritoneal space. The pumping system may be automatically activated and may also be provided with an additional voluntary control for delivering insulin at times of specific needs.

If the insulin solution is supplied by a syringe this will also usually function as the insulin reservoir. Devices of the syringe type are generally carried extracorporeally. However, considerably more sophisticated systems have also been developed, in which the entire mechanical unit is constructed for implantation, usually subcutaneously. The insulin reservoir will usually be adapted for percutaneous refilling.

The propensity of insulin to precipitate out of commercially available solutions, thereby obstructing both mechanical parts and delivering catheters, has proved to constitute a major impediment to further development and safer clinical application of continuous infusion equipment. Furthermore, there are obvious reasons for endeavouring to decrease the size of any type of continuous delivery system, whereby a need is created for more concentrated insulin solutions than have been available heretofore, which again may further aggravate the above mentioned problems.

It is generally assumed that the explanation of the precipitation phenomenon has to be sought in the tendency of insulin to form insoluble fibrils, particularly when solutions thereof are kept at elevated temperatures for extended periods of time. There is also evidence to show that any type of motion of or in the solution, including for example the turbulence induced by its passage through a narrow lumen or orifice, induces insulin fibrillation. Evidently, insulin solutions are subjected to most or all of such actions in any type of continuous delivery equipment. The general shortcomings of prior art insulin preparations in this respect are amply documented in the literature, for example in a recent review article by W. D. Lougheed et al. (Diabetologia vol. *19* (1980) pp. 1—9).

To solve this problem it has been proposed to use acid insulin solutions or neutral insulin formulations containing a sugar, such as glucose (D. S. Schade et al.: Satelite-Symposium to 16th European Association for the Study of Diabetes-Meeting, Greece, 22—23 Sept., 1980, p. 107), or a synthetic nonionic surfactant (H. Thurow: German Patent Appln. P 29 52 119.5).

However, insulin is chemically unstable in acid, even below body temperature and may react reversibly or irreversibly, with carbohydrates. Further, the above-mentioned non-physiological surfactant could be regarded as undesirable in drugs for parenteral use.

Such inconveniences are overcome according to the present invention which devises novel insulin solutions in which the insulin is substantially less prone to precipitate under conditions prevailing in continuous insulin delivery equipment than has been the case with conventional insulin preparations.

The invention is based on the discovery that calcium or magnesium ions at certain concentrations may exert a stabilizing effect on insulin solutions. This is a surprising observation because calcium in analogy with a number of divalent metal ions is generally known to form slightly soluble complexes with insulin, and to decrease the solubility of zinc insulin at neutral pH (J. E. Pitts et al. Insulin, Chemistry, Structure and Function of Insulin and Related Hormones, p 674 (Walther de Gruyter & Co. 1980); S. O. Emdin et al.: Diabetologia 19 (1980) pp. 174—182).

The present invention provides an insulin preparation comprising zinc insulin in aqueous solution and a preserving agent, the pH of the preparation being in the range of from 7 to 8, characterised in that the preparation comprises calcium or magnesium in essentially ionized form at a concentration in the range of from $0.4 \times 10^{-3}$ molar to $10^{-2}$ molar, the concentration being sufficient to impart the necessary physical stability to the insulin preparation in an insulin delivery system but insufficient to induce insulin precipitation therein under normal conditions.

In a second aspect of the present invention there is also provided a method for making an insulin preparation suitable for use in insulin delivery systems by being physically stable under conditions prevailing therein, which method is characterized by bringing insulin into aqueous solution together with calcium or magnesium in essentially ionizable form in an amount sufficient to provide in the insulin preparation a concentration of calcium or magnesium in the range of from $0.4 \times 10^{-3}$ to $10^{-2}$ molar and at a concentration sufficient to impart the necessary physical stability to said solution, but insufficient to induce insulin precipitation therein under normal conditions; further adding a preserving agent, the pH of the preparation made being in the range of from 7 to 8. Optionally, a non-ionizing osmotic pressure regulating agent and/or a pH buffering agent which does not form complex or slightly soluble compounds with calcium or magnesium ions may be added.

Preferred embodiments of the invention and detailed description thereof.

Generally, the stability of insulin solutions (as measured by the test method to be described hereinafter) increases with increasing concentrations of $Ca^{++}$ or $Mg^{++}$ up to a point where almost instantaneous precipitation of insulin may occur. At ambient temperature the upper limit of the non-precipitating concentration of $Ca^{++}$ or $Mg^{++}$ decreases from about $10^{-2}$ molar to about $3\times10^{-3}$ molar over the concentration interval of insulin corresponding to from 5 to 50 international units (I.U.) per ml, and to about $2\times10^{-3}$ molar over the concentration interval corresponding to from 50 to 1000 I.U. per ml.

In a preferred embodiment of the present invention the molarity of calcium or magnesium ions in the preparation does not exceed those respective limits, and is preferably not less than about $0.4\times10^{-3}$. Preferred are calcium or magnesium salts of inorganic acids, such as hydrochloric, sulphuric and nitric acid, the calcium salts being most preferred.

Preferably, the total molar concentration of other metal salts than those of calcium or magnesium, such as sodium salts, does not exceed 0.01, and the presence of calcium or magnesium precipitating or complexing anions, such as phosphate, gluconate, citrate or acetate, is preferably avoided. Hence if isotonicity and buffering capacity of the insulin solution is desired, the former is preferably established by means of a non-electrolyte, such as glycerol, and the latter by means of a non-complexing buffer, such as TRIS.

Another preferred embodiment involves the use of porcine, bovine, or human insulin, of which the zinc content does not exceed 1 per cent by weight (calculated on the basis of dry insulin crystals). The concentration of insulin in the solution is preferably in the range of from 5 to 1,500 I.U., more preferably from 40 to 1,000 I.U., per ml.

An exemplary mode of preparing the insulin solutions of the present invention comprises dissolving crystalline zinc insulin, for example a highly purified grade of insulin, such as "monocomponent" insulin (see British Patent No. 1,285,023) in water in the presence of acid, for example hydrochloric acid. An aqueous solution of the preserving agent, for example phenol or an alkyl phenol, such as cresol, or methyl parahydroxybenzoate, is prepared separately, if desired also containing an osmotic pressure regulating agent, such as glycerol, preferably in an amount calculated to render the final solution isotonic. This solution is then added to the acid insulin solution followed by addition of a base, for example sodium hydroxide solution, to adjust the pH to neutrality. In this specification neutrality is to be understood as a pH-value in the range of from about 7 to about 8. The calculated amounts of calcium or magnesium salt, such as calcium chloride dihydrate, or magnesium chloride hexahydrate, and buffering agent (if desired), such as TRIS, may be added at this stage, followed by readjustment of pH. Alternatively, the calcium or magnesium salt may be dissolved in the acid insulin solution prior to neutralization thereof. The resulting solution is finally filled up to the calculated volume with water, sterilized by filtration and subsequently transferred to sterile vials.

The present invention also provides a method of infusing insulin in humans, characterized in that the insulin preparation used therefor is an insulin preparation according to claim 1.

Stability test

The insulin solutions so prepared are subjected to a stability test under forced conditions in the following manner:

Vials (of 12.5 ml capacity) containing the test sample (10 ml) and each provided with a rubber cap are placed vertically on a shaking platform (Type 01 T623TBSH02, supplied by HETO, Birkerφd, Denmark) which is totally immersed in a water bath kept at 41°C±0.1°C. The platform is subjected to horizontal rocking movements with a frequency and amplitude of 100 rpm and 50 mm, respectively.

The opalescence of the test samples is monitored at regular time intervals on a Fischer DRT 1000 nephelometer provided with an adapter for vials. Fibrillation time is defined as the lapse of time until the test sample develops a turbidity of 10 nephelometric turbidity units (NTU).

Each test is conducted with test samples and control samples without added calcium or magnesium salt (4—5 vials of each) treated side by side. The stability factor is calculated as the ratio of the average fibrillation time of the test samples to that of the control samples.

Further details of practising the present invention are furnished by way of the following Examples.

In the Examples, aqueous solutions and water were sterilized, the former by filtration, and subsequent operations thereof were conducted under aseptic conditions.

Example 1
500 I.U. insulin per ml in $2\times10^{-3}$ molar solution of calcium chloride.

Crystalline monocomponent porcine insulin (7.44 g) containing 0.4 per cent of zinc and having a total activity of 200.000 I.U. was dissolved in water (250 ml) containing hydrochloric acid (6.5 ml of 1 N), followed by the addition of an aqueous solution (100 ml) containing glycerol (6.4 g) and phenol (0.8 g). The pH of the solution was adjusted to 7.5 by means of sodium hydroxide solution, and the total volume to 400 ml with water. To an aliquot of this solution (100 ml) was added 29.4 mg calcium chloride dihydrate and the resulting solution was sterilized by filtration and subsequently transferred aseptically to vials (10 ml).

Stability factor: >25.

## Example 2

500 I.U. insulin per ml in $3 \times 10^{-3}$ and 0.02 molar solution of calcium chloride and TRIS, respectively.

Crystalline monocomponent porcine insulin (3.72 g) containing 0.4% zinc and having a total activity of 100.000 I.U. was dissolved in water (125 ml) containing hydrochloric acid (3.25 ml of 1 N), followed by the addition of aqueous solution (50 ml) containing glycerol (3.2 g) and phenol (0.4 g). The pH was adjusted to 7.5 by means of sodium hydroxide solution. TRIS (0.48 g) and calcium chloride (88 mg of dihydrate) were added. The pH was readjusted to 7.5 with hydrochloric acid and the volume made up to a total of 200 ml with water. The resulting solution was sterilized by filtration and subsequently transferred aseptically to vials (10 ml).

Stability factor: 5.1.

## Example 3

500 I.U. insulin per ml in $0.5 \times 10^{-3}$ molar solution of calcium chloride.

Crystalline monocomponent porcine insulin (5.58 g) containing 0.4 per cent zinc and having a total activity of 150.000 I.U. was dissolved in water (150 ml) containing hydrochloric acid (4.9 ml of 1 N) and calcium chloride 22 mg of dihydrate) followed by the addition of an aqueous solution (100 ml) containing glycerol (4.8 g) and phenol (0.6 g).

The pH of the solution was adjusted to 7.5 by means of sodium hydroxide solution and the total volume to 300 ml with water. The resulting solution was sterilized by filtration and subsequently transferred aseptically to vials (10 ml).

Stability factor: 2.3.

## Example 4

500 I.U. insulin per ml in $2 \times 10^{-3}$ molar solution of calcium chloride.

The procedure was analogous to that of Example 3, except that 88 mg of calcium chloride dihydrate was added thus affording a solution containing 0.002 M calcium chloride.

Stability factor: >25.

## Example 5

200 I.U. insulin per ml in $2 \times 10^{-3}$ molar solution of calcium chloride.

300 ml of an insulin solution prepared according to example 4 was diluted with 450 ml of a neutral aqueous solution containing glycerol (7.2 g), phenol (0.9 g) and calcium chloride (132 mg of dihydrate). The resulting insulin solution containing 200 I.U./ml was sterilized by filtration and transferred aseptically to vials (10 ml).

Stability factor: 12.

## Example 6

40 I.U. insulin per ml in $2 \times 10^{-3}$ molar solution of calcium chloride.

Crystalline monocomponent porcine insulin containing 0.4 per cent of zinc and having a total activity of 40.000 I.U. was dissolved in 120 ml water containing hydrochloric acid (1280 µl of 1 N) followed by addition of an aqueous solution (730 ml) of phenol (2 g) and glycerol (16 g). An aqueous solution (40 ml) containing sodium hydroxide (2000 µl of N) was added, followed by addition of an aqueous solution (15 ml) containing calcium chloride (220 mg of dihydrate). Finally the pH was adjusted to 7.4 by means of sodium hydroxide solution, and the total volume to 1000 ml with water. The solution was sterilized by filtration and subsequently transferred aseptically to vials (10 ml).

Stability factor: 3.

## Example 7

500 I.U. insulin per ml in $(2 \times 10^{-3})$ molar solution of magnesium chloride.

Crystalline monocomponent porcine insulin (3.72 g) containing 0.4% zinc and having a total activity of 100.000 I.U. was dissolved in water (125 ml) containing hydrochloric acid (3.25 ml of 1 N), followed by the addition of an aqueous solution (50 ml) containing glycerol (3.2 g) and phenol (0.4 g). The pH was adjusted to 7.5 by means of sodium hydroxide solution. Magnesium chloride (81 mg of hexahydrate) was added, pH readjusted to 7.5 with sodium hydroxide and the volume made up to a total of 200 ml with water. The resulting solution was sterilized by filtration and subsequently transferred aseptically to vials (10 ml).

Stability factor: 6.

## Example 8

100 I.U. insulin per ml in $(2 \times 10^{-3})$ molar solution of magnesium chloride.

Crystalline monocomponent porcine insulin (3.72 g) containing 0.4% zinc and having a total activity of 100.000 I.U. was dissolved in water (125 ml) containing hydrochloric acid (3.25 ml of 1 N), followed by the addition of an aqueous solution (800 ml) containing glycerol (16 g) and phenol (2 g). The pH was adjusted to 7.5 by means of sodium hydroxide solution. Magnesium chloride (407 mg of hexahydrate) was added, pH readjusted to 7.5 and the volume made up to a total of 1000 ml with water. The resulting solution was sterilized by filtration and subsequently transferred aseptically to vials (10 ml).

Stability factor: 4.

## Example 9

100 I.U. human insulin per ml in $2 \times 10^{-3}$ molar solution of calcium chloride.

Crystalline semisynthetic human insulin, prepared by the method described in British Patent Application No. 2 069 502A, (2.04 g) containing 0.4% zinc and having a total activity of 55.000 I.U. was dissolved in water (275 ml) containing hydrochloric acid (1.8 ml of 1 N). To an aliquot of this solution (25 ml) was added calcium chloride dihydrate (14.7 mg) followed by the addition of an aqueous solution (20 ml) containing glycerol (0.8 g) and phenol (0.1 g). The pH of the solution was adjusted to 7.4 by means of sodium hydroxide solution and the total volume to 50 ml with water.

The resulting solution was sterilized by filtration and subsequently transferred aseptically to vials (10 ml.

Stability factor: 7.

## Claims

1. An insulin preparation comprising zinc insulin in aqueous solution and a preserving agent, the pH of the preparation being in the range of from 7 to 8, characterised in that the preparation comprises calcium or magnesium in essentially ionized form at a concentration in the range of from $0.4 \times 10^{-3}$ molar to $10^{-2}$ molar, the concentration being sufficient to impart the necessary physical stability to the insulin preparation in an insulin delivery system but insufficient to induce insulin precipitation therein under normal conditions.

2. An insulin preparation according to Claim 1, which further comprises a non-ionizing osmotic pressure controlling agent and/or a pH buffering agent which does not form complex or slightly soluble compounds with calcium or magnesium ions.

3. An insulin preparation according to Claim 1 or 2, wherein the concentration of calcium or magnesium in ionized form does not exceed the approximate molarities of $10^{-2}$, $3 \times 10^{-3}$ and $2 \times 10^{-3}$ for insulin concentrations corresponding to 5, 50 and 1000 international insulin units per ml, respectively.

4. An insulin preparation according to any one of the preceding claims, wherein the calcium or magnesium is furnished by a salt of hydrochloric, sulphuric or nitric acid.

5. An insulin preparation according to any one of the preceding claims, comprising calcium in essentially ionized form.

6. An insulin preparation according to any one of the preceding claims, wherein the insulin concentration is in the range corresponding to from 5 to 1500 international insulin units per ml.

7. An insulin preparation according to Claim 6, wherein the insulin concentration is in the range corresponding to from 40 to 1000 international insulin units per ml.

8. A method for making an insulin preparation, which method is characterized by bringing zinc insulin into aqueous solution together with calcium or magnesium in essentially ionizable form in an amount sufficient to provide in the insulin preparation a concentration of calcium or magnesium in the range of from $0.4 \times 10^{-3}$ molar to $10^{-2}$ molar and at a concentration sufficient to impart the necessary physical stability to the insulin preparation in an insulin delivery system but insufficient to induce insulin precipitation therein under normal conditions; further adding a preserving agent, the pH of the preparation made being in the range of from 7 to 8.

9. A method according to Claim 8, wherein there is further added a non-ionizing osmotic pressure regulating agent and/or a pH buffering agent which does not form complex or slightly soluble compounds with calcium or magnesium ions.

10. A device for continuous insulin delivery, which comprises an insulin reservoir, a pumping system and a catheter for delivering insulin, the reservoir containing an insulin preparation in accordance with any one of Claims 1 to 7, and/or made by the method of Claims 8 or 9.

## Patentansprüche

1. Insulinzubereitung, enthaltend Zink-Insulin in wässriger Lösung sowie ein Konservierungsmittel, wobei das pH des Präparats im Bereich von 7 bis 8 liegt, dadurch gekennzeichnet, dass die Zubereitung Calcium oder Magnesium in im wesentlichen ionisierter Form bei einer Konzentration im Bereich von $0,4 \times 10^{-3}$ Molar bis $10^{-2}$ Molar umfasst, wobei die Konzentration genügend ist, um der Insulinzubereitung in einem Insulin-Verabreichungssystem die nötige physikalische Stabilität zu verleihen, jedoch ungenügend ist, um darin unter normalen Bedingungen die Ausfällung von Insulin zu verursachen.

2. Insulinzubereitung nach Anspruch 1, ferner enthaltend ein nichtionisierendes Agens zur Einstellung des osmotischen Drucks und/oder ein das pH puffernde Agens, das mit Calcium- oder Magnesiumionen weder komplexe noch nur wenig lösliche Verbindungen bildet.

3. Insulinzubereitung nach Anspruch 1 oder 2, in welcher die Konzentration des in ionisierter Form vorliegenden Calciums oder Magnesiums annähernd die Molaritäten $10^{-2}$, $3 \times 10^{-3}$ bzw. $2 \times 10^{-2}$ für Insulinkonzentrationen von 5, 50 bzw. 1000 Internationalen Insulineinheiten pro ml nicht überschreitet.

4. Insulinzubereitung nach einem der vorangehenden Ansprüche, in welcher das Calcium oder Magnesium von einem Salz der Salzsäure, Schwefelsäure oder Salpetersäure geliefert wird.

5. Insulinzubereitung nach einem der vorangehenden Ansprüche, enthaltend Calcium in im wesentlichen ionisierter Form.

6. Insulinzubereitung nach einem der vorangehenden Ansprüche, in welcher die Insulinkonzentration im Bereich von 5 bis 1500 Internationalen Insulineinheiten pro ml liegt.

7. Insulinzubereitung nach Anspruch 6, in welcher die Insulinkonzentration im Bereich von 40 bis 1000 Internationalen Insulineinheiten pro ml liegt.

8. Verfahren zur Herstellung einer Insulinzubereitung, gekennzeichnet durch das Lösen von Zink-Insulin in wässrige Lösung zusammen mit Calcium oder Magnesium in im wesentlichen ionisierter Form in genügender Menge, um in der Insulinzubereitung eine Konzentration an Calcium oder Magnesium im Bereich von $0,4 \times 10^{-3}$ Molar bis $10^{-2}$ Molar zu ergeben und in einer Konzentration, die genügend ist, um der Insulinzubereitung in einem Insulin-Verabreichungssystem die nötige physikalische Stabilität zu verleihen, jedoch ungenügend ist, um darin unter normalen Bedindungen die Ausfällung von Insulin zu verursachen; ferner durch das Beigeben eines Konservierungsmittels, um das pH der Zubereitung im Bereich von 7 bis 8 einzustellen.

9. Verfahren nach Anspruch 8, beim welchem auch noch ein nichtionisierendes Agens zur Einstellung des osmotischen Drucks und/oder ein das pH puffernde Agens, das mit Calcium- oder Magnesiumionen weder komplexe noch nur wenig lösliche Verbindungen bildet, beigegeben wird.

10. Vorrichtung zur kontinuierlichen Verabreichung von Insulin, umfassend einen Insulinbehälter, ein Pumpsystem und ein Katheter zur Verabreichung von Insulin, wobei der Behälter eine nach einem der Ansprüche 1 bis 7 definierte und/oder nach einem der Ansprüche 8 oder 9 hergestellte Insulinzubereitung enthält.

**Revendications**

1. Préparation d'insuline contenant l'insuline de zinc en solution aqueuse et un agent de conservation, préparation dont le pH est compris dans une plage de 7 à 8, caractérisée en ce que la préparation contient du calcium ou du magnésium essentiellement sous forme ionisée, à une concentration dans une plage de $0,4\times10^{-3}$ molaires à $10^{-2}$ molaires, la concentration étant suffisante pour conférer la stabilité physique nécessaire à la préparation d'insuline dans un système de distribution d'insuline mais insuffisante pour provoquer la précipitation d'insuline dans celui-ci dans des conditions normales.

2. Préparation d'insuline selon la revendication 1 contenant de plus un agent non-ionisant de régulation de la pression osmotique et/ou un agent tampon de pH qui ne forme pas de composés complexes ou légèrement solubles avec les ions calcium ou magnésium.

3. Préparation d'insuline selon la revendication 1 ou 2, dans laquelle la concentration en calcium ou en magnésium sous forme ionisée n'excède pas les molarités approximatives de $10^{-2}$, $3\times10^{-3}$ et $2\times10^{-3}$ pour des concentrations en insuline correspondant respectivement à 5, 50 et 1000 unités internationales d'insuline par ml.

4. Préparation d'insuline selon l'une quelconque des revendications précédentes dans laquelle le calcium ou le magnésium est fourni sous forme d'un sel d'acide chlorhydrique, sulfurique ou nitrique.

5. Préparation d'insuline selon l'une quelconque des revendications précédentes contenant du calcium essentiellement sous forme ionisée.

6. Préparation d'insuline selon l'une quelconque des revendications précédentes dans laquelle la concentration en insuline est dans une plage correspondant à une valeur de 5 à 1500 unités internationales d'insuline par ml.

7. Préparation d'insuline selon la revendication 6 dans laquelle la concentration en insuline est dans une plage correspondant à une valeur de 40 à 1000 unités internationales d'insuline par ml.

8. Procédé pour réaliser une préparation d'insuline, ce procédé étant caractérisé en ce qu'on amène de l'insuline de zinc en solution aqueuse, ensemble avec du calcium ou du magnésium essentiellement sous forme ionisable en une quantité suffisante pour obtenir une préparation d'insuline à une concentration de calcium ou de magnésium dans une plage de $0,4\times10^{-3}$ molaires à $10^{-2}$ molaires et à une concentration suffisante pour conférer la stabilité physique nécessaire à la préparation d'insuline dans une système de distribution d'insuline mais insuffisante pour provoquer la précipitation d'insuline dans celui-ci dans des conditions normales et en ce qu'on ajoute un agent de conservation, le pH de la préparation obtenue se trouvant dans une plage de 7 à 8.

9. Procédé selon la revendication 8 caractérisé en ce qu'on ajoute de plus un agent non-ionisant de régulation de la pression osmotique et/ou un agent tampon de pH qui ne forme pas de composés complexes ou légèrement solubles avec les ions calcium ou magnésium.

10. Dispositif pour la distribution continue d'insuline qui comporte un réservoir d'insuline, un système de pompage et un cathéter pour distribuer l'insuline, le réservoir contenant une préparation d'insuline selon l'une quelconque des revendications 1 à 7 et/ou préparée par le procédé des revendications 8 ou 9.